# EUROPEAN PATENT SPECIFICATION

(11) **EP 1 680 501 B1**
(45) Date of publication and mention of the grant of the patent: **19.12.2012**
(21) Application number: 04810630.6
(22) Date of filing: 08.11.2004
(51) Int. Cl.: C12N 1/04

(54) **STABILIZED COMPOSITIONS COMPRISING PROBIOTICS**
PROBIOTIKA ENTHALTENDE STABILISIERTE ZUSAMMENSETZUNGEN
COMPOSITIONS STABILISEES COMPRENANT DES PROBIOTIQUES

(30) Priority: 07.11.2003 US 704253
(43) Date of publication of application: 19.07.2006
(73) Proprietor: THE PROCTER & GAMBLE COMPANY, Cincinnati, OH 45202 (US); Alimentary Health Limited, Kinsale, County Cork (IE)
(72) Inventor: MYATT, Graham John, Camberley, Surrey GU166GE (GB); CHARBONNEAU, Duane Larry, Mason, Ohio 45040 (US); WRIGHT, Kevin, Ian, Trevor, Frimley Green, Surrey GU166JZ (GB); HALLISSEY, Martin, Laleham, Middlesex TW181RX (GB)
(74) Representative: Canonici, Jean-Jacques
(86) International application number: PCT/US2004/037428
(87) International publication number: WO 2005/047489

(56) References cited:
- EP-A- 0 298 605
- EP-A- 0 904 784
- EP-A- 1 344 458
- WO-A-96/08261
- WO-A-98/10666
- DE-A1- 19 819 475
- US-A1- 2004 175 389
- SAARELA MARIA ET AL: "Probiotic bacteria: Safety, functional and technological properties" JOURNAL OF BIOTECHNOLOGY, vol. 84, no. 3, 28 December 2000 (2000-12-28), pages 197-215, XP002317975 ISSN: 0168-1656

## Description

### Technical Field

The present invention relates to stabilised dry bacterial compositions having a low water activity. The compositions herein have long-term stability and probiotic activity.

### Background

Recently, probiotics and compositions comprising these materials have become increasingly popular for the treatment of many ailments. Probiotics can be bacteria, or purified fractions thereof, that provide a benefit, such as disease relief or prophylaxis, to a host following consumption. Whilst many varieties of probiotic bacteria exist, compositions comprising these materials, particularly viable probiotic bacterial cells, tend to have poor stability. For example, dried concentrates of probiotic bacteria have been administered to mammals in milk and other aqueous suspensions. However, unless these compositions are stored and distributed under refrigerated conditions, it has previously been necessary to prepare the suspension immediately prior to use from a dried concentrate, or to consume the dried concentrate itself in powder or capsule form, in order to ensure that a sufficiently high percentage of the cells administered remain viable at the time of administration.

Whilst it is recognised that dried bacterial concentrates provide some stability benefits, these have not provided entirely suitable stability and ease of use. A major problem concerning probiotic-containing compositions is the level of water available in the composition. Moderate to high levels of water in probiotic-containing compositions comprising dried bacteria concentrates enable the dried bacteria to continue metabolising during storage. This metabolism results in the production of acidic metabolites and other molecules, as well as the breakdown and reduction in viability of the probiotic bacteria themselves, that render the composition "off", or tainted and therefore not fit for consumption or efficacious. A variety of excipients, and similar suspension materials have been pursued in an attempt to lock away water in probiotic-containing compositions, all with varying degrees of success. For example US 4,518,696 discloses a stabilised liquid bacterial composition consisting of a mixture of dried viable cells of animal-probiotic Lactobacilli and fumed silica, the mixture having a water activity of less than 0.20, dispersed in anhydrous sunflower seed oil. Despite these advances, long-term storage stability of dry bacterial compositions has been far from optimised, even for the most stable of bacterial strains. Several bacterial strains still require storage at 5°C or below, and even then, long-term stability is not guaranteed.

Therefore, a need exists for improved probiotic compositions, having improved stability and increased delivery of viable probiotic bacteria. In particular, a need exists for providing stable probiotic compositions comprising bacterial strains that have previously been very difficult to store long-term at room temperature.

### Summary

The present invention provides unit dose dry bacterial compositions as set out in Claim 1. The compositions have improved long-term stability at both 5°C and room temperature in bulk powder, encapsulated forms or other like forms. The present invention also provides packaged dry bacterial compositions and methods of manufacturing the compositions of the present invention.

### Brief Description of Figures

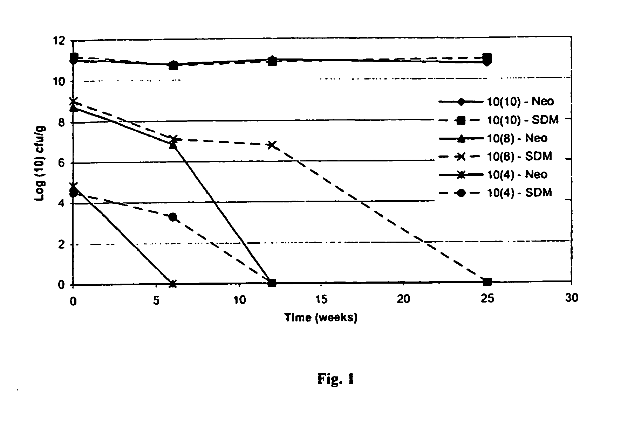

Figure 1: Dependence of storage stability at 25°C over time on starting bacterial concentration measured as colony forming units per gram (cfu/g) of a 50/50 mix of bacteria at 2 x 10¹⁰, 2 x 10⁸ and 2 x 10⁴ cfu/g with either Mannogem EZ (SDM) or Neosorb 20/60 (Neo).

### Detailed Description

All weights, measurements and concentrations herein are measured at 25°C on the composition in its entirety, unless otherwise specified.

Unless otherwise indicated, all percentages of compositions referred to herein are weight percentages and all ratios are weight ratios.

Unless otherwise indicated, all molecular weights are weight average molecular weights.

As used herein, the abbreviation "cfu/g" means "colony forming units per gram", as measured using the method provided as part of the European Pharmacopoeial Methods, 2003, Section 2.6.12.

As used herein "dry bacteria compositions" includes compositions comprising less than 20% materials that are liquid at room temperature, preferably less than 10%, more preferably less than 8%, more preferably still less than 6% by weight of the total composition.

As used herein "bound water" means water molecules that are tightly held by various chemical groups in larger molecules such as carboxyl, hydroxyl and amino groups.

The present invention provides dry bacterial compositions having a water activity (a measure of the ability of bound water to de-sorb from the molecule) of less than 0.5. Preferably, the water activity of the compositions of the present invention is less than 0.4, more preferably less than 0.25, more preferably still less than 0.15. Even more preferably, the water activity of the compositions according to the present invention is less than 0.1. Water activity can be determined using methods known to those skilled in the art. Herein, water activity is determined using a NovaSina TH200 Water Activity Meter at 25°C. Briefly, the meter is calibrated using calibration salts. The sample to be measured is temperature equilibrated in the meter, following which the water activity is determined as the percent relative humidity (%RH) divided by 100 after equilibrium is reached (typically 10 to 20 minutes).

It has been found that by reducing the water activity of dry bacterial compositions, their stability can be improved. Without being limited by theory, it has surprisingly been found that the water activity of the compositions of the present invention can be decreased, and hence the stability of the composition increased, by increasing the concentration (number of viable cells) of the dried bacteria concentrate, and the proportion (by weight of total composition) of the dried bacteria concentrate in the composition, relative to other constituents. Again, without limitation, it is believed that, in combination with a stabiliser having a low water content and low water activity, the compositions of the present invention have less water content in total, and what water is present in the composition is tightly bound to its carrier molecules.

Without being limited by theory, it is believed that the dried bacteria concentrate can be viewed as an amorphous solid that has a glass transition temperature (T_{g}) that affects the stability of the system. The T_{g} determines the phase transition of a composition from the kinetically stable solid, glass-like phase, to the thermodynamically stable liquid/rubbery state. For storage stability, the kinetically stable phase (i.e. the glass phase) is preferred as reaction rates and diffusion rates are much lower than in the liquid/rubbery phase. Furthermore, it has been recognised that bound water molecules are more easily de-sorbed and used in biochemical metabolism in the liquid/rubbery state. The water activity and content of a system inversely impacts the T_{g}; the higher the water activity or content, the lower the T_{g}. Therefore, by decreasing the water activity or content of the system, the T_{g} is increased, and the stability of the system itself is increased. Therefore controlling the contribution of the dried bacteria concentrate and any filler materials to the overall water activity, and therefore T_{g} of the composition can improve the stability of the composition as a whole. It has surprisingly been found that the dried bacteria themselves have a low water activity. Therefore, it has been found that using a high level (i.e. at least 10% by weight of the dry bacteria composition) of the dried bacterial concentrate, wherein the concentrate has a high concentration of bacteria, stabilises the compositions by keeping the water content and water activity low when compared with compositions comprising either lower amounts of dried bacterial concentrate, or concentrates having lower bacteria counts.

Furthermore, it is preferable that the total dry bacteria composition comprising the dried bacteria concentrate is anhydrous. As used herein, "anhydrous" means that the composition has a water content of less than 20%. Without being bound by theory, it is believed that in conjunction with the low water activity, the dry bacterial compositions of the present invention having a water content of less than 20% have improved stability due to the low level of water available in the composition, and the fact that what water is present in the composition is tightly bound to its carrier molecules. Water content can be determined using methods known to those skilled in the art. Herein, water content is determined using a TGA Thermal Gravimetric Analyser from TA Instruments and associated software. The analyser method is set to equilibrate at room temperature (25°C) followed by a linear ramp increase in temperature at 20°C per minute to a final temperature of 105°C, followed by a 20-minute hold at 105°C. The data is analysed using the accompanying analysis programme supplied with the analyser, and the water content of the sample determined as a percent of the sample mass. More preferably the dry bacterial compositions of the present invention have a water content of less than 10%, more preferably still less than 8%.

Without being bound by theory, it is believed that, by using a high level of dried bacteria concentrate, the dried bacterial concentrate can act as a large reservoir to bind any water that is available in the composition. In so doing, any water present in the composition is dispersed through the large amount of dried bacteria concentrate, thereby not depressing the T_{g} of the composition sufficiently to pass to the less stable liquid/rubbery state at the storage temperature, and thus maintaining the stability of the composition.

The compositions of the present invention comprise at least 10% by weight of the total composition of a dried bacteria concentrate, preferably at least 30%, more preferably at least 50%. As used herein, the term "dried bacteria concentrate" includes fermentation cultures of bacteria that have been concentrated by a process such as centrifugation, freeze-drying, spray drying or combinations thereof known to those skilled in the art, to yield a dried concentrated bacterial product containing a high number of bacterial cells that can be added to the composition of the present invention. The dried bacteria concentrate comprises bacteria at levels of 1 x 10¹⁰ to 1 x 10¹⁴ cfu/g before being added to the composition of the present invention. The bacteria present in the dried bacterial concentrate may be viable (i.e. "alive") or killed cultures of bacteria. Preferably the bacteria present in the concentrate are viable. As used herein, the term "viable" means that at least 50% of the bacteria present are capable of colony formation using standard bacterial plating methods known to those skilled in the art, preferably at least 60%, more preferably at least 75% and more preferably still at least 90%.

In order to determine the level of dried bacteria concentrate, and the concentration of bacteria therein, methods known to those skilled in the art may be employed. For example, in order to determine the amount of dried bacterial concentrate present in a dry bacteria composition, the composition could be dissolved with mixing in a known volume of a suitable diluent such as phosphate buffered saline. An initial microscopic evaluation can then be carried out at a suitable dilution to assess the state of the material. Bacterial enumeration techniques known to those skilled in the art may be used, such as the standard plate count method, fluorescent techniques such as flow cytometry and the D-count method, Neubauer counter enumeration (otherwise known as a haemocytometer) in conjunction with stains such as crystal violet or phase contrast microscopy. The relative proportions of dried bacteria concentrate to other materials present in the dry bacterial composition may be evaluated by subtracting the mass of excipient, stabiliser or other materials from the total dry weight of the composition. Such materials (i.e. the non-dried bacteria concentrate) may be separated using a variety of techniques known to those skilled in the art. For example, soluble materials may be dissolved and then filtered or centrifuged, and the supernatant subsequently dried and the dry mass weighed. Insoluble materials may be separated by density gradient centrifugation as known to those skilled in the art. Depending upon the formulation, the skilled person will choose those methods that result in the correct and accurate determination of the concentration and level of dried bacteria concentrate present in the composition.

The dried bacteria concentrate may comprise other materials such as nutrients, bacterial excretions and other soluble material present in the fermentation cultures of the bacteria prior to drying. Preferably these materials are present at levels of less than 20%, more preferably less than 10% by weight of the dried bacteria concentrate. Furthermore, in order to increase the concentrations of bacteria in the dried bacterial concentrate, it may be preferable to centrifuge or filter the growth media containing the bacteria prior to drying, to separate the bacteria from the media. By removing the majority of the liquid prior to drying, the majority of the soluble nutrients and materials will be removed, and therefore will not be present in the dried bacteria concentrate. This is desirable to increase the relative proportion of bacteria in the concentrate, and also to avoid excessive contamination of the dried bacteria concentrate with any bacterial toxins or other such materials that may not be suitable for consumption by mammals.

The bacteria may be grown as a pure (single strain) or mixed (multiple strains) culture of the desired bacteria in a liquid medium which gives satisfactory growth of the culture(s) involved. Such a medium may be composed of protein or protein fractions, various fermentable carbohydrates, growth stimulants, inorganic salts, buffers etc; or the medium may be sterile whole milk, skim milk, whey, or other natural substrates, or combinations thereof. After inoculation, the culture is allowed to develop under generally optimised incubation conditions of time and temperature. Depending on the organism(s) being grown, the incubation times may range from periods of 4 to 48 hours, and the temperatures may vary from 15°C to 50°C. It may also be desirable to control pH and dissolved oxygen. After satisfactory growth has been attained, the culture in its growth medium is cooled to between 0° to 15°C.

In general, the method used for obtaining dried bacteria concentrate is carried out in accordance with known procedures for culturing such bacteria. After a satisfactory bacterial population has been attained in a suitable growth medium, the pH of the broth may be lower than desirable for preparing a dried product. Typically, the final pH will range from 4.4 to 5.4. Before drying of the fermentation broth, it is advantageous to add an alkaline reagent, such as sodium hydroxide to adjust the pH upwardly to a pH more favourable to the stability of the bacteria. In general, as previously known, it is desirable to adjust the pH upwardly toward neutrality (pH 7), the adjustment being at least to pH 5.8. Any food-acceptable alkali can be used [NaOH, KOH, NH4 OH, Ca(OH)₂, etc.]. Adjustment to a pH of about 6.0 to 6.5 is preferred. By way of specific example, the pH may be raised by the addition of sodium hydroxide to a pH of about 6.2. Where other additives are to be incorporated in the growth medium which will effect its pH, such as the stability potentiators of this invention, the pH adjustment can be made last as a matter of convenience.

Where the bacterial concentrate is dried by freeze-drying, it may be desirable to incorporate a cryoprotectant in the fermentation culture before drying. Suitable known cryoprotectants include inositol, sorbitol, mannitol, glucose, sucrose, corn syrup, DMSO, starches and modified starches of all types, PVP, maltose, or other mono and disaccharides. The level of addition can range from 1.0 to 300 grams per liter of culture depending on the particular agent. An effective amount should be used to minimize cell damage on freezing. Furthermore, the dried bacteria concentrate needs to be dried sufficiently to lower the water content to less than 20%, preferably less than 10%, more preferably less than 8%, more preferably still less than 6%. It is desirable to select a cryoprotectant such that the dried bacteria concentrate has a low water activity, preferably less than 0.5. Where a different method of drying is employed, such as a heat drying procedure, the cryoprotectant will not be used, and in general, any of the various procedures for drying bacteria or servitive biological materials to a powder can be used. These include freeze-drying, spray drying, roller and/or vacuum pan drying. In practicing the present invention, the preferred drying procedures are freeze-drying or spray drying.

The dried bacteria concentrate may comprise any bacterial family, genus, species or strain that is not harmful to host animals upon oral consumption, preferably those bacterial strains that are not harmful, preferably a probiotic, following oral consumption in mammals, more preferably following oral consumption in humans or companion animals. As indicated above, bacteria may produce toxins and other molecules that may be harmful to mammals, particularly humans. Whilst any bacteria may be stabilised in the composition of the present invention, it is preferable that the composition is suitable for consumption by mammals. Preferably, the bacteria comprise lactic acid bacteria. Non-limiting examples of lactic acid bacteria suitable for use herein include strains of *Streptococcus lactis, Streptococcus cremoris, Streptococcus diacetylactis. Streptococcus thermophilus, Lactobacillus bulgaricus, Lactobacillus acidophilus, Lactobacillus helveticus, Lactobacillus bifidus, Lactobacillus casei, Lactobacillus lactis, Lactobacillus plantarum, Lactobacillus rhamnosus, Lactobacillus delbruekii, Lactobacillus thermophilus, Lactobacillus fermentii, Lactobacillus salivarius, Bifidobacterium longum, Bifidobacterium infantis, Bifidobacterium bifidum,* and *Pediococcus cerevisiae,* or mixtures thereof, preferably *Lactobacillus salivarius, Bifidobacterium infantis,* or mixtures thereof.

As a non-limiting example, strains of *Bifidobacterium* isolated from resected and washed human gastrointestinal tract as disclosed in WO 00/42168 are preferred. More preferred is the *Bifidobacterium infantis* strain designated UCC35624, described as being deposited at the National Collections of Industrial and Marine Bacteria Ltd (NCIMB) on January 13, 1999, and accorded the accession number NCIMB 41003.

As another non-limiting example, strains of *Lactobacillus salivarius* isolated from resected and washed human gastrointestinal tract as described in WO 98/35014 are preferred. More preferred are the *Lactobacillus salivarius* strains that are designated UCC 1 and UCC 118, described as being deposited at the National Collections of Industrial and Marine Bacteria Ltd (NCIMB) on November 27, 1996, and accorded the accession numbers NCIMB 40830 and 40829, respectively.

### Optional Components

The dried bacterial compositions of the present invention further comprise a stabiliser. Preferably, the dry bacteria composition comprises a combination of high levels of dried bacteria concentrate and a stabiliser that has both a low water content, and a low water activity, the overall Tg of the system is maintained as high as possible, thereby rendering the composition more stable. Stabilisers are useful in the present invention to act as stabilising fillers or bulking agents whilst not increasing the water activity or content of the system suffciently to reduce the stability of the system. The stabiliser of the present invention comprises a material or materials having a water activity of less than 0.5 when at a water content of 10%. Preferably, the stabiliser has a water activity of less than 0.4, more preferably less than 0.25, more preferably still less than 0.15. Preferably the water content of the stabiliser is less than 10%, more preferably less than 8%, more preferably still less than 6%. Where the composition is to be encapsulated, the stabiliser preferably has a water activity of less than 0.4, more preferably less than 0.15, and a water content of less than 8%, more preferably less than 6%. Without wishing to be bound by theory, this is believed to be due to the fact that the encapsulation process may introduce further water into the composition, when compared with the dried bulk composition alone, and therefore the composition prior to encapsulation needs to be as dry as possible.

The compositions of the present invention comprise from 1% to 90% stabiliser by weight of the composition, more preferably from 10% to 70% stabiliser, more preferably still from 20% to 50% stabiliser.

The stabiliser of the present invention may comprise any material that has a water content and water activity as defined above. Preferably, the stabiliser is a flowable solid. By flowable solid is meant a material that is a particulate solid having a Carr's index of less than 20%, preferably less than 15%. As used herein, Carr's index is determined using ASTM Designation D6393-99; "Standard Test Method for Bulk Solids Characterization by Carr Indices" (2002). Preferably, at least one stabiliser is selected from the group comprising polysaccharides, oligosaccharides, disaccharides, cellulose-based materials, polyols, polyhydric alcohols, silicas, zeolites, clays, aluminas, starches, sugars, or mixtures thereof, more preferably polysaccharides, oligosaccharides, cellulose-based materials, silicas, zeolites, clays, aluminas, starches, sugars, or mixtures thereof. More preferably still, at least one stabiliser is selected from the group comprising polysaccharides, cellulose-based materials, starches, or mixtures thereof. Non-limiting examples of materials suitable for use in the present invention are set out in table 1.

| **Material** | **Tradename/Supplier** | **Water Activity** | **Water Content (%)** |
|---|---|---|---|
| Microcrystalline Cellulose | Avicel ph 112 - FMC | 0.04 | 1.5 |
| Psyllium Hemicellulose | Psyllium | 0.05 | 8 |
| Potato Starch | Supplied by Avebe America inc | 0.09 | 4 |
| Maltodextrin | Maltodextrin - A.E. Stanley | 0.25 | 5 |

**Table 1: Non-limiting examples of materials suitable for use as a stabiliser in the compositions of the present invention.**

| | | | |
|---|---|---|---|
| Spray Dried Mannitol | Mannogem EZ - SPI Pharma | 0.36 | <0.5 |
| Sucrose | Supplied by Particel Control Inc. | 0.36 | <0.1 |
| Sorbitol | Neosorb 20/60 - Roquette | 0.39 | <2.0 |
| Magnesium stearate | Supplied by Peter Greven | 0.41 | <6.0 |
| Mannitol | Pearlitol 500DC - Stobec Inc. | 0.42 | <0.5 |
| Sucralose | Supplied by McNeil | 0.42 | <2.0 |
| Sorbitol | Neosorb P35/60 - Roquette | 0.43 | <2.0 |
| Xylitol | Xylitol - Roquette | 0.44 | <1_{.}0 |
| Microcrystalline Cellulose | Avicel ph 302 - FMC | 0.44 | <5.0 |
| Maltitol | Maltisorb p90 - Roquette | 0.46 | <1.0 |
| Isomalt | Isomalt DC 100 - Palatinit | 0.48 | <1.5 |

Preferably, the stabiliser itself has a glass transition temperature (T_{g}) at a water content of 10% of greater than 273K, preferably greater than 288K, more preferably greater than 293K. As used herein, glass transition temperature is determined using ASTM E1356-98 "Standard Test Method for Assignment of the Glass Transition Temperatures by Differential Scanning Calorimetry or Differential Thermal Analysis" (2003).

Referring to figure 1., it can be seen that the starting concentration of the dried bacteria concentrate severely impacts the stability of the dry composition over time at room temperature (25°C). The stability of compositions comprising 50% of a 1 x 10⁴ cfu/g dried bacteria concentrate have severely limited storage stability, when compared with those comprising 50% of either a 1 x 10⁸ or 1 x 10¹⁰ dried bacteria concentrate. Furthermore, the affect of the water activity and water content of the stabiliser is evidently demonstrated by the stability data. The mannogem EZ (SDM) has a water activity of 0.36 and water content of less than 0.5%, compared with the water activity and content of Neosorb 20/60 (0.39 and less than 2.0% respectively).

The compositions of the present invention may be in the form of a packaged composition. The stabiliser may be added to the composition if necessary, at any time during processing, prior to packaging. The stabiliser may be added to the fermentation broth prior to drying, or mixed with the dried bacterial concentrate as a powder, following which the composition is subsequently packaged. Where the stabiliser is added to the bacterial fermentation broth, it may be added during fermentation, immediately prior to drying or after a concentrating process such as centrifugation, or at a variety of these stages during processing. Preferably, the stabiliser is dry mixed as a powder with the dried bacterial concentrate.

Where the dry bacteria composition is in the form of a packaged composition, the composition may be in the form of a bulk powder, packaged in sealed containers such as jars or sachets, or may be encapsulated using methods known to those skilled in the art. Where the composition is encapsulated, the coating preferably comprises low water content materials. Non-limiting examples of suitable encapsulation materials include hydroxypropylmethylcellulose, gelatin, starches, alginates or mixtures thereof, preferably hydroxypropylmethylcellulose. Types and methods of encapsulation are well known to those skilled in the art. Other methods are described in WO 2004/030652.

The compositions of the present invention may, independently, comprise additional optional components to enhance their performance. For example, one or more vitamins, enzymes, plasticizers, coloring agents, flavoring agents, sweeteners, anti-oxidants, buffering agents, slip aids, other excipients, and the like can be optionally included in the compositions herein. Non-limiting examples of optional components are given below.

An optional ingredient suitable for use herein includes vitamins. For example, vitamin A, vitamin B₁, vitamin B₂, vitamin B₆, vitamin B₁₂, niacin, folic acid, biotin, vitamin C, vitamin D, vitamin E, vitamin K, and mixtures thereof may be used. Fat-soluble vitamins, for example beta-carotene and other source of vitamin A, may be particularly useful for inclusion due to their sensitivity to moisture. Vitamin C, vitamin E, and mixtures thereof are also particularly useful.

Another example of optional components includes one or more enzymes. For example, a proteolytic enzyme (e.g., pancreatin) may be utilized.

One or more pigments or other suitable coloring agents, such as dyes and lakes, may be incorporated into the compositions. U.S. FD&C dyes (e.g., yellow #5, blue #2, red # 40) and / or U.S. FD&C lakes are may be used. Preferred lakes which may be used in the present invention include, for example, Lake red #40, yellow #6, blue #1, and the like. Additionally, a mixture of U.S. FD&C dyes and / or U.S. FD&C lakes in combination with other conventional food and food colorants may be used. As further examples, Riboflavin and β-carotene may also be used. Additionally, other natural coloring agents may be utilized including, for example, fruit, vegetable, and / or plant extracts such as grape, black currant, aronia, carrot, beetroot, red cabbage, and hibiscus. The amount of coloring agent used will vary, depending on the agents used and the character or intensity desired in the finished composition. One of ordinary skill in the art will readily make such determination.

One or more flavouring agents may be incorporated in the compositions of the present invention in order to enhance their palatability. Any natural or synthetic flavour agent can be used in the present invention. As used herein, such flavours may be synthetic or natural flavours.

For example, one or more botanical and / or fruit flavours may be utilized herein. Particularly preferred fruit flavours are exotic and lactonic flavours such as, for example, passion fruit flavours, mango flavours, pineapple flavours, cupuacu flavours, guava flavours, cocoa flavours, papaya flavours, peach flavours, and apricot flavours. Besides these flavours, a variety of other fruit flavours can be utilized such as, for example, apple flavours, citrus flavours, grape flavours, raspberry flavours, cranberry flavours, cherry flavours, grapefruit flavours, and the like. These fruit flavours can be derived from natural sources such as fruit juices and flavour oils, or may alternatively be synthetically prepared. The amount of flavouring agent used will vary, depending on the agents used and the character or intensity desired in the finished composition. One of ordinary skill in the art will readily make such determination.

One or more sweeteners, including for example carbohydrate sweeteners and natural and /or artificial no / low calorie sweeteners may optionally be used herein. For example, the compositions of the present invention can be sweetened with any of the carbohydrate sweeteners, preferably monosaccharides and / or disaccharides. Preferred sugar sweeteners for use in the compositions of the present invention are sucrose, fructose, glucose, maltose, and mixtures thereof.

One or more high intensity sweeteners may be utilized. For example, one or more of the following sweeteners may be utilized: saccharin, cyclamates, L-aspartyl-L-phenylalanine lower alkyl ester sweeteners (e.g., aspartame); L-aspartyl-D-alanine amides disclosed in U.S. Patent No. 4,411,925; L-aspartyl-D-serine amides disclosed in U.S. Patent No. 4,399,163; L-aspartyl-L-I-hydroxymethylalkancamide sweeteners disclosed in U.S. Patent No. 4,338,346; L-aspartyl-1-hydroxyethyalkaneamide sweeteners disclosed in U.S. Patent No. 4,423,029; L-aspartyl-D-phenylglycine ester and amide sweeteners disclosed in European Patent Application 168, 112; N-[N-3,3-dimethylbutyl)-L-alpha-aspartyl)-L-phenylalanine 1-methyl ester sweeteners disclosed in WO 99/30576; thaumatin; dihydrochalcones; cyclamates; steviosides; glycyrrhizins, synthetic alkoxy aromatics; sucralose; suosan; miraculin; monellin; sorbitol, xylitol; talin; cyclohexylsulfamates; substituted imidazolines; synthetic sulfamic acids such as acesulfame, acesulfame K and n-substituted sulfamic acids; oximes such as perilartine; peptides such as aspartyl malonates and succanilic acids; dipeptides; amino acid based sweeteners such as gem-diaminoalkanes, *meta*-aminobenzoic acid, L-aminodicarboxylic acid alkanes, and amides of certain alpha-aminodicarboxylic acids and gem-diamines; and 3-hydroxy-4-alkyloxyphenyl aliphatic carboxylates or heterocyclic aromatic carboxylates; erythritol; and mixtures thereof. Aspartame is particularly preferred. The amount of sweetener used will vary, depending on the agents used and the character or intensity desired in the finished composition. One of ordinary skill in the art will readily make such determination.

One or more anti-oxidants may be utilized in the compositions of the present invention. Naturally occurring as well as synthetic anti-oxidants may be used. Non-limiting examples of natural anti-oxidants include tocopherols (e.g., vitamin E), ascorbic acid (e.g., vitamin C), vitamin A (e.g., beta-carotene), grape seed extract, selenium, and coenzyme Q10. Non-limiting examples of synthetic anti-oxidants include butylated hydroxytoluene (BHT), butylated hydroxyanisole (BHA), and propyl gallate.

Other non-limiting examples of optional components useful in the compositions of the present invention include diclofenac, naproxen, aspirin, indomethacin, omeprazole, cardiac glycosides, electrolyte preparations with sodium, potassium, or magnesium salts as well as calcium and iron preparations, bisacodyl preparations, valproic acid, 5-ASA, steroids such as hydrocortisone, budesonide, laxatives, octreotide, cisapride, anticholinergies, calcium channel blockers, 5HT3-antagonists such as ondansetron and peptides such as insulin.

Non-limiting examples of excipients include sweeteners (such as described herein below); flavour and/or colouring agents (such as described herein below), solid lubricants, such as stearic acid and magnesium stearate; calcium sulfate; vegetable oils, such as peanut oil, cottonseed oil, sesame oil, olive oil, corn oil and oil of theobroma; emulsifiers, such as TWEENS; wetting agents, such as sodium lauryl sulfate; tabletting agents such as binders, antioxidants; and preservatives.

### Method of Manufacture

Due to the sensitivity of the compositions of the present invention to water levels and oxygen, it is preferable to control the levels of these materials during the manufacturing process. As such, it has been found that the atmosphere under which the compositions of the present invention are dried, milled, mixed and packaged should preferably have a relative humidity (RH) of less than 50%, preferably less than 40%, more preferably less than 36%. In addition, it is preferable that the compositions are prepared under a low oxygen atmosphere. As used herein a "low oxygen atmosphere" includes atmospheres comprising less than 10% oxygen, preferably less than 8% oxygen. Low oxygen atmospheres can be generated using an inert atmosphere such as nitrogen, so as to displace any oxygen present in the final composition. Low oxygen atmospheres are desirable as any oxygen present in the compositions may result in oxidative degradation, and subsequent loss of bacterial viability, and the composition becoming tainted, or "off".

Furthermore, it is may be desirable to pre-condition commercially available stabilisers to reduce their water content still further, prior to mixing with bacteria. Non-limiting examples of how this can be achieved include oven drying under reduced pressure (vacuum), freeze-drying, water scavenging by desiccants, and fluid bed drying.

### Method of Use

The compositions of the present invention are intended to be used as a prophylactic, therapeutic treatment or non-therapeutic treatment to alleviate diseases and conditions that affect animals, preferably mammals, preferably humans. Non-limiting elements of animal health and physiology that benefit, either in therapeutically relieving the symptoms of, or disease prevention by prophylaxis include inflammatory disorders, immunodeficiency, inflammatory bowel disease, irritable bowel syndrome, cancer (particularly those of the gastrointestinal and immune systems), diarrhoeal disease, antibiotic associated diarrhoea, appendicitis, autoimmune disorders, multiple sclerosis, Alzheimer's disease, rheumatoid arthritis, diabetes mellitus, bacterial infections, viral infections, fungal infections, periodontal disease, urogenital disease, surgical associated trauma, surgical-induced metastatic disease, sepsis, weight loss, anorexia, fever control, cachexia, wound healing, ulcers, gut barrier infection, allergy, asthma, respiratory disorders, circulatory disorders, coronary heart disease, anaemia, disorders of the blood coagulation system, renal disease, disorders of the central nervous system, hepatic disease, ischaemia, nutritional disorders, osteoporosis, endocrine disorders, and epidermal disorders. Preferred are treatment of the gastrointestinal tract, including treatment or prevention of diarrhoea; immune system regulation, preferably the treatment or prevention of autoimmune disease and inflammation; maintaining or improving the health of the skin, preferably treating or preventing atopic disease of the skin; ameliorating or reducing the effects of aging, including mental awareness and activity levels; and preventing weight loss during and following infection. The diarrhoeal diseases may be associated with gastrointestinal inflammatory activity.

Typically, the compositions of the present invention are given to an individual as part of a dose regimen. The dose regime is dependent upon the dosing format used in which the dry bacteria composition is incorporated. Unit dose forms have been described above as either capsule or sachet form. Typically, the unit dose provides the individual with bacteria at a level of from 1 x 10⁵ cfu per dose to 1 x 10¹⁵ cfu per dose, preferably from 1 x10⁷ cfu to 1 x10¹⁴ cfu per dose. The unit dose, when provided as a capsule can be swallowed directly. When provided as a sachet filled with the dry bacteria composition, the powder may be ingested directly, or mixed with milk, yoghurt, or other liquid carrier materials. Typically, capsules may provide lower dosing amounts than sachets, as the size of the capsule, and its relative easy of ingestion, will limit the amount of dry bacteria composition that can be filled therein. Preferably, the unit dose is taken by the individual at least once per month, preferably at least once a week, more preferably at least once per day.

### Examples:

The following examples further describe and demonstrate embodiments within the scope of the present invention. They are given for the purpose of illustration and are not to be construed as limitations of the present invention. Where applicable, ingredients are given in CTFA name.

| **Ex.** | **Material** | **Water Activity** | **Water Content (%)** | **Weight (%)** |
|---|---|---|---|---|
| 1 | Freeze Dried *B*. *Infantis* (5x10¹² CFU/g) | 0.04 | 6 | 50 |
| | Microcrystalline Cellulose | 0.04 | <1.5 | 50 |
| 2 | Freeze Dried *B. Infantis* (1x10¹⁰ CFU/g) | 0.04 | 6 | 25 |
| | Potato Starch | 0.09 | <6 | 75 |
| 3 | Freeze Dried *B. Infantis* (1x10¹¹ CFU/g) | 0.04 | 6 | 40 |
| | Psyllium hemicellulose | 0.05 | <8 | 60 |
| 4 | Freeze Dried L. *Salivarius* (5x10¹² CFU/g) | 0.04 | 5 | 80 |
| | Microcrystalline Cellulose | 0.04 | 1 | 20 |
| 5 | Freeze Dried *L. Acidophilus* (3x10¹¹ CFU/g) | 0.04 | 5 | 60 |
| | Maltodextrin | 0.25 | 5 | 39.5 |
| | Magnesium Stearate | 0.41 | <6 | 0.5 |
| 6 | Freeze Dried *B. Infantis* (1x10¹¹ CFU/g) | 0.04 | 6 | 45 |
| | Potato Starch | 0.09 | <6 | 39.25 |
| | Magnesium Stearate | 0.41 | <6 | 0.75 |
| | Ascorbic Acid | - | | 15 |
| 7 | Freeze Dried *B. Infantis* (2x10¹² CFU/g) | 0.04 | 6 | 15 |
| | Freeze Dried L. *Salivarius* (2x 10¹² CFU/g) | 0.04 | 5 | 15 |
| | Microcrystalline Cellulose | 0.04 | <1.5 | 27 |
| | Fumed Silica | - | - | 2 |
| | Magnesium Stearate | 0.41 | <6 | 1 |
| | Ascorbic Acid | - | - | 20 |
| | Tricalcium citrate | - | - | 20 |
| 8 | Freeze Dried *B. Infantis* (5x10¹¹ CFU/g) | 0.04 | 6 | 30 |
| | Freeze Dried *L*. *Salivarius* (5x10¹¹ CFU/g) | 0.04 | 5 | 30 |
| | Microcrystalline Cellulose | 0.04 | <1.5 | 23 |
| | Fumed Silica | - | - | 1 |
| | Magnesium Stearate | 0.41 | <6 | 1 |
| | Ascorbic Acid | - | - | 5 |
| | Calcium lactate gluconate | - | - | 10 |

The above examples are dry bacteria compositions prepared according to the following procedure. All operations are performed in a humidity-controlled environment where the RH is maintained between 30 and 36%. The appropriate amount of freeze-dried bacteria (pre-concentrated to the desired CFU/g) are added to the mixing cavity of a Pharmatech mixer along with the appropriate amount of stabiliser such as microcrystalline cellulose, potato starch or the like. The bacterial and stabilisers have been chosen for their low water activity and low water content as well as similar particle size and densities to allow for more efficient mixing. The head space within the mixing cavity is flushed with dry Nitrogen gas such that the gasses of the original headspace have been replaced a total of 10 times or until the RH inside the mixing cavity is reduced to below 20%. The mixing cavity is then sealed with an airtight lid and the powders mixed together for 20 minutes at a rotation speed of 60rpm. Once mixing has finished the stability of the powder blend can be maintained by ensuring the powders are not exposed to high RH's (greater than 36% RH) or water-rich environments. The dry-blended powders can be packaged into gelatin capsules under a nitrogen/low RH environment and stored in sealed containers or as dry powders in sachets or containers. The resulting capsules and powders contained therein have improved long-term stability both at low temperatures (4°C) and room temperature (25°C).

In a further embodiment, the dry bacteria compositions of examples 1 to 8 can be packaged into unit dose forms such as capsules or sachets under a nitrogen/low (<36%) relative humidity (RH) environment. Examples 9 to 11 demonstrate non-limiting examples of unit dose compositions packaged in and packaged into capsules. The capsules are intended to be taken as a single dose, swallowed whole. Examples 12 to 14 are non-limiting examples of unit dose compositions packaged into sachets, providing higher bacteria counts per dose when compared with the capsules.

| **Example** | **Packaging Format** | **Dry Bacteria Composition** | **CFU per dose** |
|---|---|---|---|
| **9** | Gelatin Capsule | 100 mag of Ex. 1 | 2.5 x 10¹¹ |
| **10** | HPMC Capsule | 180 mg of Ex. 2 | 4.5 x 10⁸ |
| **11** | Gelatin Capsule | 250 mg of Ex. 5 | 1 x 10¹² |
| **12** | Sachet | 2 g of Ex. 7 | 1.2 x 10¹² |
| **13** | Sachet | 5 g of Ex. 8 | 1.5 x 10¹² |
| **14** | Sachet | 1 g of ex. 4 | 4 x 10¹² |

## Claims

1. A unit dose composition, for administration to a human by swallowing, comprising;
a) a capsule containing a dry bacterial composition having a water activity of less than 0.5 and providing from 1 x 10⁵ cfu to 1 x 10¹⁵ cfu of bacteria per dose;
b) the dry bacterial composition comprising:
i) at least 10% by weight of the total bacterial composition of a dried bacteria concentrate; and
ii) from 1% to 90% of a stabilizer having a water activity of less than 0.5 at a water content of 10%;
**characterized in that** the concentration of bacteria in the dried bacteria concentrate is from 1 x 10¹⁰ cfu/g to 1 x 10¹⁴ cfu/g_{.}

2. The unit dose composition according to Claim 1 wherein the dry bacterial composition has a water activity of less than 0.4, preferably less than 0.25.

3. The unit dose composition according to Claim 1 comprising at least 30% of said dried bacterial concentrate.

4. The unit dose composition according to Claim 1 wherein the dry bacterial composition has a total water content of less than 20%.

5. The unit dose composition according to Claim 1 wherein said stabiliser has a glass transition temperature at a water content of 10% of greater than 273K.

6. The unit dose composition according to Claim 1 wherein the stabiliser is selected from polysaccharides, oligosaccharides, disaccharides, cellulose-based materials, polyols, polyhydric alcohols, silicas, zeolites, clays, aluminas, starches, sugars, and mixtures thereof.

7. The unit dose composition according to Claim 1 where said dried bacterial concentrate comprises lactic acid bacteria of the genus *Streptococci, Lactobacillus, Bifidobacteria,* or mixtures thereof.

8. The unit dose composition according to Claim 7 wherein said lactic acid bacteria comprise bacteria of the species *Lactobacillus salivarius, Bifidobacterium infantis,* or mixtures thereof.

9. The unit dose composition according to Claim 1 wherein the capsule comprises hydroxypropylmethylcellulose, gelatin, starch, alginates, or mixtures thereof.

## Patentansprüche

1. Einheitsdosenzusammensetzung zur oralen Verabreichung an einen Menschen, die Folgendes umfasst:
a) eine Kapsel, die eine Trockenbakterienzusammensetzung mit einer Wasseraktivität von weniger als 0,5 enthält und 1 x 10⁵ cfu bis 1 x 10¹⁵ cfu Bakterien pro Dosis ergibt;
b) wobei die Trockenbakterienzusammensetzung Folgendes umfasst:
i) zu mindestens 10 Gew.-% der Gesamtbakterienzusammensetzung ein Trockenbakterienkonzentrat; und
ii) zu 1 % bis 90 % ein Stabilisierungsmittel mit einer Wasseraktivität von weniger als 0,5 bei einem Wassergehalt von 10%;
**dadurch gekennzeichnet, dass** die Konzentration an Bakterien im Trockenbakterienkonzentrat 1 x 10¹⁰ cfu/g bis 1 x 10¹⁴ cfu/g beträgt.

2. Einheitsdosenzusammensetzung nach Anspruch 1, wobei die Trockenbakterienzusammensetzung eine Wasseraktivität von weniger als 0,4, vorzugsweise von weniger als 0,25 aufweist.

3. Einheitsdosenzusammensetzung nach Anspruch 1, die zu mindestens 30 % das Trockenbakterienkonzentrat umfasst.

4. Einheitsdosenzusammensetzung nach Anspruch 1, wobei die Trockenbakterienzusammensetzung einen Gesamtwassergehalt von weniger als 20 % aufweist.

5. Einheitsdosenzusammensetzung nach Anspruch 1, wobei das Stabilisierungsmittel eine Glasumwandlungstemperatur bei einem Wassergehalt von 10 % von mehr als -15 °C (273 K) aufweist.

6. Einheitsdosenzusammensetzung nach Anspruch 1, wobei das Stabilisierungsmittel ausgewählt ist aus Polysacchariden, Oligosacchariden, Disacchariden, cellulosebasierten Materialien, Polyolen, mehreren Hydroxylgruppen enthaltenden Alkoholen, Kieselsäuren, Zeoliten, Tonerden, Aluminiumoxiden, Stärken, Zuckern und Mischungen davon

7. Einheitsdosenzusammensetzung nach Anspruch 1, wobei das Trockenbakterienkonzentrat Milchsäurebakterien der Gattung *Streptococci, Lactobacillus, Bifidobacteria* oder Mischungen davon umfasst.

8. Einheitsdosenzusammensetzung nach Anspruch 7, wobei die Milchsäurebakterien Bakterien der Species *Lactobacillus salivarius, Bifidobacterium infantis* oder Mischungen davon umfassen.

9. Einheitsdosenzusammensetzung nach Anspruch 1, wobei die Kapsel Hydroxypropylmethylcellulose, Gelatine, Stärke, Alginate oder Mischungen davon umfasst.

## Revendications

1. Composition en dose unitaire, pour administration à un humain par ingestion, comprenant :
a) une gélule contenant une composition de bactéries sèche ayant une activité à l'eau inférieure à 0,5 et fournissant de 1 x 10⁵ UFC à 1 x 10¹⁵ UFC de bactéries par dose;
b) la composition bactérienne sèche comprenant:
i) au moins 10 % en poids de la composition bactérienne totale d'un concentré de bactéries séché; et
ii) de 1 % à 90 % d'un agent stabilisant ayant une activité à l'eau inférieure à 0,5 à une teneur en eau de 10 % ;
**caractérisée en ce que** la concentration de bactéries dans le concentré de bactéries séché va de 1 x 10¹⁰ UFC/g à 1x 10¹⁴ UFC/g.

2. Composition en dose unitaire selon la revendication 1, dans laquelle la composition bactérienne sèche a une activité à l'eau inférieure à 0,4, de préférence inférieure à 0,25.

3. Composition en dose unitaire selon la revendication 1, comprenant au moins 30 %, dudit concentré bactérien séché.

4. Composition en dose unitaire selon la revendication 1, dans laquelle la composition bactérienne sèche a une teneur totale en eau inférieure à 20 %.

5. Composition en dose unitaire selon la revendication 1, dans laquelle ledit agent stabilisant a une température de transition vitreuse à une teneur en eau de 10 % supérieure à -15 °C (273 K).

6. Composition en dose unitaire selon la revendication 1, dans laquelle l'agent stabilisant est choisi parmi les polysaccharides, oligosaccharides, disaccharides, matériaux à base de cellulose, polyols, alcools polyhydriques, silices, zéolites, argiles, alumines, amidons, sucres, et leurs mélanges.

7. Composition en dose unitaire selon la revendication 1, où ledit concentré bactérien séché comprend des bactéries d'acide lactique du genre *Streptococci, Lactobacillus, Bifidobacteria,* et leurs mélanges.

8. Composition en dose unitaire selon la revendication 7, dans laquelle lesdites bactéries d'acide lactique comprennent des bactéries de l'espèce *Lactobacillus salivarius, Bifidobacterium infantis,* et leurs mélanges.

9. Composition en dose unitaire selon la revendication 1, dans laquelle la gélule comprend de l'hydroxypropylméthylcellulose, de la gélatine, de l'amidon, des alginates, ou leurs mélanges.
